# EUROPEAN PATENT APPLICATION

(11) **EP 2 466 306 A1**
(43) Date of publication of application: **20.06.2012**
(21) Application number: 10195545.8
(22) Date of filing: 17.12.2010
(51) Int. Cl.: G01N 33/497, A61B 5/083, G01N 1/22

(54) **Holder for a liquid separator and gas analyzer for analyzing respiratory gas samples**

(71) Applicant: General Electric Company, Schenectady, NY 12345 (US)
(72) Inventor: Viitala, Juha, 01710, Vantaa (FI); Hakanen, Jukka, 02750, Espoo (FI); Varis, Anu, 01450, Vantaa (FI); Hyytiäinen, Eero, 00510, Helsinki (FI); Karlsson, Kai, 00660, Helsinki (FI); Kauppi, Jani, 00250, Helsinki (FI)
(74) Representative: Kanerva, Arto

(57) **Abstract**

A holder for a liquid separator (1) is disclosed herein. The liquid separator includes a separator body (9) for dividing a sample flow into two separate flows, one of said flows substantially free of liquid being for the gas measurement and another of said flows including at least one of liquid and gas bypassing the measurement, and which liquid separator also includes a detachable liquid container (10) for receiving the another flow. The holder includes a space (21) receiving the liquid separator and also includes a frame (22) surrounding at least partly the space and which frame including a first contact point (23) being in contact with the separator body. The frame also includes a second contact point (24) being in contact with the liquid container and which first contact point together with the second contact point are adapted to hold the liquid separator.

## Description

### BACKGROUND OF THE INVENTION

The disclosure relates generally to a holder for a liquid separator, the liquid separator comprising a separator body for dividing a respiratory gas sample flow into two separate flows, one of said flows substantially free of liquid being for the gas measurement and another of said flows including at least one of liquid and gas bypassing said gas measurement, and which liquid separator also comprising a detachable liquid container for receiving said another flow including liquid. The disclosure also relates to a gas analyzer for analyzing respiratory gas samples.

In anesthesia or in intensive care, a condition of a patient is often monitored e.g. by analyzing a gas exhaled by the patient for its content. For this reason a small portion of the respiratory gas may be diverted to a gas analyzer. This sample often carries along to the analyzer some water vapor, which condensates into droplets, and also some dust, water, mucus and blood. Such components carried along with the sample would have a detrimental effect on the gas analyzer and measuring result. This is why the dust and fluid components must be removed from a gas sample upstream of the actual gas analyzer. In this so called the sidestream configuration, a liquid separator, also called a water trap, is located in series with the sampling line to separate water, mucus and other unwanted material from the gas sample.

According to a well-known principle a gas sample is picked up from the exhalation air of a patient to delivered into the water trap or especially into a first passage of this water trap, from which the water component along with a minor amount of gas is sucked away, usually by way of a liquid receiver or container. Most of the gas flow received in the first passage is sucked through a gas permeable and fluid impermeable material into the second passage and further to the gas analyzer. This fluid impermeable hydrophobic material effectively prevents the passage of water to the gas analyzer.

In many water traps, the container with collected water can be taken apart from the water trap for emptying. Typically an upper part of the water trap comprising the first and second passages and also the gas permeable and fluid impermeable material is fixed to the gas analyzer while a lower part of the water trap, which is the container, is detachably fixed to the upper part, because it is usually emptied when it is full of liquid and then again fixed to the upper part of the water trap. If the container is not correctly in place, the sampled gas is diluted with room air typically withdrawn by a pump through the open pneumatic connection for the cup. This leads to severely incorrect measured gas concentrations. In the worst case, because of incorrect concentration results hypoxic gas mixture may be given to the patient without any alarms issued by the respiratory monitor.

In practice, detection of the presence of the water container is difficult. Simple pneumatic detection is hardly possible because of the very variable pressure conditions in the gas sampling system. A mechanical micro switch is used in the prior art, but it is expensive and suffers from reliability problems caused by water or mucus getting into the micro switch. Optical detection methods also can be used, but its reliability may be compromised by water, mucus or ambient light. All the methods mentioned have the drawback that gas leaks trough the connection between the container and the water trap remains undetected even if the presence of the container could be detected.

### A BRIEF DESCRIPTION OF THE INVENTION

The above-mentioned shortcomings, disadvantages and problems are addressed herein which will be understood by reading and understanding the following specification.

In an embodiment, a holder for a liquid separator, the liquid separator including a separator body for dividing a respiratory sample flow into two separate flows, one of the flows substantially free of liquid being for the gas measurement and another of the flows including at least one of liquid and gas bypassing the gas measurement, and which liquid separator also including a detachable liquid container for receiving the another flow. The holder including a space for receiving the liquid separator. The holder for a liquid separator also includes a frame surrounding at least partly the space and which frame includes a first contact point adapted to be in contact with the separator body. The frame also includes a second contact point adapted to be in contact with the liquid container and which first contact point together with the second contact point are adapted to hold the liquid separator.

In another embodiment, a gas analyzer for analyzing respiratory gas samples includes a gas sensor for measuring at least one gas component and a gas withdrawing apparatus for withdrawing respiratory gas samples to the gas sensor. The gas analyzer for analyzing respiratory gas samples also includes a holder for a liquid separator, the liquid separator including a separator body for dividing the respiratory gas sample flow into two separate flows, one of the flows substantially free of liquid being for the gas measurement and another of the flows including at least one of liquid and gas bypassing the gas measurement, and which liquid separator also including a detachable liquid container for receiving the another flow including at least one of liquid and gas. The holder includes besides a space for receiving the liquid separator but also a frame surrounding at least partly the space and which frame includes a first contact point adapted to be in contact with the separator body of the liquid separator. The frame also includes a second contact point adapted to be in contact with the liquid container of the liquid separator and which first contact point together with the second contact point are adapted to hold the liquid separator.

In yet another embodiment a gas analyzer for analyzing respiratory gas samples includes a gas sensor for measuring at least one gas component and a gas withdrawing apparatus for withdrawing respiratory gas samples to the gas sensor. The gas analyzer for analyzing respiratory gas samples also includes a liquid separator including a separator body for dividing the respiratory gas sample flow into two separate flows, one of the flows substantially free of liquid being guided to the gas sensor and another of the flows including at least one of liquid and gas bypassing the gas sensor, and which liquid separator also including a detachable liquid container for receiving the another flow and for collecting liquid. The gas analyzer for analyzing respiratory gas samples further includes a holder for the liquid separator, which holder includes a space for receiving the liquid separator and a frame surrounding at least partly the space. The frame includes a first contact point adapted to be in contact with the separator body of the liquid separator and a second contact point adapted to be in contact with the liquid container of the liquid separator and which first contact point together with the second contact point are adapted to hold the liquid separator.

Various other features, objects, and advantages of the invention will be made apparent to those skilled in art from the accompanying drawings and detailed description thereof.

### A BRIEF DESCRIPTION OF THE DRAWINGS

Fig. 1 shows the principle of a liquid separator connected to a gas analyzer.

Fig. 2 is a schematic view of a connection of a liquid separator to a gas analyzer according to one embodiment.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

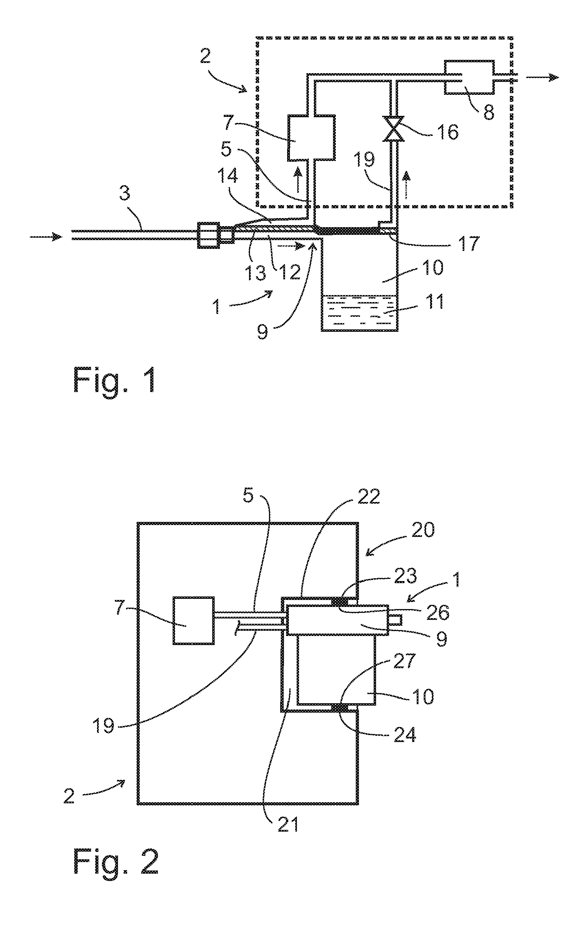

Figures 1 and 2 show a liquid separator 1 connected to a gas analyzer 2. Along a sampling tube 3 as shown in Figure 1 the gas analyzer receives a sample of a respiratory gas flowing along respiratory circuit (not shown in Figures), which may be provided between a patient and a respirator (not shown in Figures). The liquid separator 1 is downstream the gas analyzer 2. The gas analyzer comprises a gas sensor 7 for measuring at least one gas component and a gas withdrawing apparatus 8 such as a pump for withdrawing respiratory gases through the liquid separator 1 to the gas sensor 7 along a first analyzer tube 5.

The liquid separator 1 comprises at least a separator body 9 and a liquid container 10 as shown in Figure 1 and 2. The separator body 9 is for dividing the sample flow into two separate flows, one of them being typically a main flow, which is a gas flow substantially free of the liquid, guided to the gas sensor 7 and another of them being a smaller side flow including at least one of liquid and gas typically bypassing the gas sensor 7 as shown in Figure 1. The liquid container, which is detachable, is for receiving the side flow including possibly liquid 11. The liquid container may also collect liquid. The separator body of the liquid separator may also comprise an input channel 12 receiving the gas sample flowing along the sampling tube 3, but the separator body 9 may further comprise a first filter 13 separating the input channel 12 from an output channel 14. The first filter 13 is used to prevent liquid from flowing from the input channel 12 to the output channel 14 and further to the gas sensor 7. If the sample flow in the sampling tube 3 includes a liquid, normally water, this liquid will end up in the liquid container 10, which is kept at negative pressure compared to the measuring side so that its gas content does not disturb the response of the gas sensor 7. This is accomplished by sucking through the liquid container 10a small amount of gas as the side flow through a second analyzer tube 19 and a restrictor 16 directly to the gas withdrawing apparatus 8. The separator body 9 may also comprise a second filter 17 used to prevent the liquid from leaving the liquid container through this second filter with the gas component and from entering the second analyzer tube 19. This second filter 17 is normally identical to the first filter 13 and can conveniently be the same piece, only separately sealed.

Naturally the separator body can be without the output channel, the first filter and the second filter, in which case the liquid separation is made in the first channel where the liquid with the gas side flow is guided to the liquid container 10 and the main gas flow is guided to the gas sensor 7. Also it is possible that the sample flow, containing both the sampled gas and liquid drawn to or condensed in the sampling tube, is flowing in the liquid separator in a vertically oriented channel with a hydrophobic surface. On the lower end of the channel, there is an abrupt turn for the flow to vertical or upwards direction. The liquid in the incoming flow does not change its direction of movement as abruptly as gases do, and the liquids then fall through a hole on the lower tip of the turning point of the channel and fall to the liquid container without a specific gas flow through the liquid container.

The liquid container 10 should advantageously be tightly connected to the separator body 9. To improve the tightness either the separator body or the liquid container may be provided with a washer (not shown in Figures) sealing this junction between the separator body and the liquid container.

A holder 20 for the liquid separator shown in Figure 2 may be e.g. an integral part of the gas analyzer 2 or detachable from gas analyzer or be a separate component in a flow communication with the gas analyzer. The holder 20 comprises besides a space 21 receiving the liquid separator 1 but also a frame 22 surrounding at least partly the space 21. The frame 22 comprises at least a first contact point 23 to be in contact with the separator body 9 and also comprises a second contact point 24 to be in contact with the liquid container 10. The first contact point and the second contact point are able to hold the liquid separator 1 in the space 21. Advantageously the first contact point and the second contact point are adapted to press both the separator body 9 and the liquid container 10 together making sure that the liquid container is substantially hermetically connected to the separator body. As shown in Figure 2 the first contact point 23 is configured to press against the separator body 9 when the second contact point 24 is configured to press against the liquid container 10. The first and the second contact points may be opposite leaving therebetween the space 21 for the liquid separator.

In Figure 2 there is shown a very practical embodiment when the frame of the holder is part of the structure of the gas analyzer but naturally the frame and the holder can be a separate component fixable to a bar for instance. The frame can comprise for instance two opposite arms with the first and second contact points and which arms are connected together leaving therebetween the space 21 for the liquid separator. The frame comprising the opposite arms can be one single piece or comprise different pieces connected together.

The first contact point 23 and the second contact point 24 may be separate components connected or connectable to the frame 22 but as well these contact points may only be a surface of the frame 22 or its arms. A distance between the first contact point and the second contact point can be substantially equal or slightly less than the distance of corresponding liquid separator contact points such as a third contact point 26 and a fourth contact point 27. It may be advantageous to arrange the distance of the first contact point and the second contact point shorter than the distance of the corresponding separator contact points to make sure the separator body and the liquid container are tightly connected together to avoid gas leaking from surroundings to the liquid separator. In case the distance of the first contact point and the second contact point is shorter than the distance of the corresponding separator contact points the distance between the first contact point and the second contact point can be extended which may happen for instance by bending the frame of the holder or by using compressible material in those first and/or second contact points. Also it is possible that the distance between the first contact point and the second contact point is unchanging but instead the distance between the third contact point 26 and the fourth contact point 27 of the liquid separator is reduced by pressing the separator body 9 and the liquid container 10 together when assembling the liquid separator between the first contact point 23 and the second contact point 24 of the frame 22.

The first contact point 23 and the second contact point 24 of the frame 22 could also be arranged differently from Figure 2. They can be other than opposite each other provided that they are able to hold the liquid separator 1 comprising both the separator body 9 and the liquid container 10 in the space 21. Also it is advantageous if tightness between the separator body and the liquid container is achieved when the liquid separator 1 is assembled between the first contact point 23 and the second contact point 24.

One way to assemble the liquid container to the holder 20 is to push it between the first contact point and the second contact point of the frame 22. In case only one of the separator body and the liquid container is assembled between the first and the second contact points of the frame, the first contact point and the second contact point are unable to hold only one of those components releasing or detaching the liquid separator in case the separator body is unconnected to the liquid container or in case one of the separator body and the liquid container is absent, because the distance of the first contact point and the second contact point is longer than the distance of the corresponding contact points of only one of the separator body and the liquid container and which may be a clear indication to the gas analyzer 2 that the gas measurement is unready and no gas flow communication between the gas sensor 7 and the liquid separator 1 is formed. The clear indication may be the fact that the measurement results show readings from the surrounding air differing from the measurement results of the respiratory gas. The gas analyzer 2 can automatically observe this.

The mechanical interface between the liquid separator 1 and the gas analyzer 2 may facilitate to connect the liquid separator only to the gas analyzer when the liquid container 10 is attached firmly enough to the space 21. As explained hereinbefore the liquid separator 1 may be attached to the gas analyzer 2 so that it is kept in place by the pressing forces from the first contact point 23 and the second contact point 24, and the liquid container 10 is pressed against the separator body 9 so that the connection between the liquid container 10 and the separator body 9 is gas tight. In case the liquid container 10, which is detachable, and the separator body 9 are not hermetically coupled together, this assembly operation may do this because the distance between the first contact point 23 and the second contact point 24 is same or less than the corresponding contact points of the liquid separator when the separator body and the liquid container are hermetically coupled together.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A holder for a liquid separator (1), the liquid separator comprising a separator body (9) for dividing a respiratory sample flow into two separate flows, one of said flows substantially free of liquid being for the gas measurement and another of said flows including at least one of liquid and gas bypassing said gas measurement, and which liquid separator also comprising a detachable liquid container (10) for receiving said another flow, said holder comprising:
a space (21) for receiving said liquid separator;
a frame (22) surrounding at least partly said space and which frame comprising a first contact point (23) adapted to be in contact with said separator body,
**characterized in that** said frame also comprising a second contact point (24) adapted to be in contact with said liquid container and which first contact point together with said second contact point are adapted to hold the liquid separator.

2. The holder according to claim 1, **characterized in that** said first contact point and said second contact point adapted to hold said liquid separator in said space are also adapted to press both said separator body and said liquid container together making sure that said liquid container is substantially hermetically connected to said separator body.

3. The holder according to claim 1, **characterized in that** said first contact point and said second contact point adapted to hold said liquid separator in said space are also adapted to release said liquid separator in case said separator body is unconnected to said liquid container.

4. The holder according to claim 1, **characterized in that** said first contact point and said second contact point adapted to hold said liquid separator in said space are also adapted to release said liquid separator in case one of said separator body and said liquid container is absent.

5. The holder according to claim 1, **characterized in that** a distance between said first contact point and said second contact point is adapted to be substantially equal or slightly less than a distance between a corresponding third contact point (26) of said separator body and fourth contact point (27) of said liquid container when said liquid container is connected to said separator body.

6. The holder according to claim 1, **characterized in that** said first contact point is opposite said second contact point leaving therebetween said space for said liquid separator.

7. The holder according to claim 1, **characterized in that** said frame is part of a structure of a gas analyzer (2).

8. The holder according to claim 7, **characterized in that** said gas analyzer is adapted to be in flow communication along a first analyzer tube (5) and a second analyzer tube (19) to said liquid separator and through said liquid separator to a sampling tube (3) for withdrawing a gas sample from a respiratory gas.

9. A gas analyzer for analyzing respiratory gas samples comprising:
a gas sensor (7) for measuring at least one gas component;
a gas withdrawing apparatus (8) for withdrawing respiratory gas samples to said gas sensor; and
a holder (20) for a liquid separator (1), the liquid separator comprising a separator body (9) for dividing the respiratory gas sample flow into two separate flows, one of said flows substantially free of liquid being for the gas measurement and another of said flows including at least one of liquid and gas bypassing said gas measurement, and which liquid separator also comprising a detachable liquid container (10) for receiving said another flow including at least one of liquid and gas, said holder comprising besides a space (21) for receiving said liquid separator but
also a frame (22) surrounding at least partly said space and which frame comprising a first contact point (23) adapted to be in contact with said separator body of said liquid separator, **characterized in that** said frame also comprising a second contact point (24) adapted to be in contact with said liquid container of said liquid separator and which first contact point together with said second contact point are adapted to hold the liquid separator.

10. The holder according to claim 9, **characterized in that** said first contact point and said second contact point adapted to hold said liquid separator in said space are also adapted to press both said separator body and said liquid container together making sure that said liquid container is substantially hermetically connected to said separator body.

11. The holder according to claim 9, **characterized in that** said first contact point and said second contact point adapted to hold said liquid separator in said space are also adapted to release said liquid separator in case said separator body is unconnected to said liquid container.

12. The holder according to claim 9, **characterized in that** said first contact point and said second contact point adapted to hold said liquid separator in said space are also adapted to release said liquid separator in case one of said separator body and said liquid container is absent.

13. The holder according to claim 9, **characterized in that** a distance between said first contact point and said second contact point is adapted to be substantially equal or slightly less than a distance between a corresponding third contact point (26) of said separator body and fourth contact point (27) of said liquid container when said liquid container is connected to said separator body.

14. The holder according to claim 9, **characterized in that** said first contact point is opposite said second contact point leaving therebetween said space for said liquid separator.

15. The holder according to claim 9, **characterized in that** said frame is part of a structure of a gas analyzer (2).
